# EUROPÄISCHE PATENTSCHRIFT

(11) **EP 0 485 856 B1**
(45) Veröffentlichungstag und Bekanntmachung des Hinweises auf die Patenterteilung: **20.07.1994**
(21) Anmeldenummer: 91118804.3
(22) Anmeldetag: 05.11.1991
(51) Int. Cl.: C07C 313/08, C07C 319/18, C07C 323/03

(54) **Verfahren zur Herstellung von 1,1-Difluoralkansulfenylchloriden**
Process for the preparation of 1,1-difluoralkansulfenyl chlorides
Procédé pour la préparation de chlorures de 1,1-difluoroalkansulfonyle

(30) Priorität: 16.11.1990 DE 4036515
(43) Veröffentlichungstag der Anmeldung: 20.05.1992
(73) Patentinhaber: BAYER AG, 51368 Leverkusen (DE)
(72) Erfinder: Böhm, Stefan, Dr., W-5090 Leverkusen (DE); Marhold, Albrecht, Dr., W-5090 Leverkusen (DE); Bielefeldt, Dietmar, Dr., W-4030 Ratingen 6 (DE)

(56) Entgegenhaltungen:
- EP-A- 0 417 558
- DE-B- 1 125 915
- DE-B- 1 220 852

## Beschreibung

Die vorliegende Erfindung betrifft ein neues Verfahren zur Herstellung von 1,1-Difluoralkansulfenylchloriden, welche als Zwischenprodukte für organische Synthesen verwendet werden können.

Es wurde gefunden, daß man die 1,1-Difluoralkansulfenylchloride der allgemeinen Formel (I)

R-CH₂-CF₂-S-Cl (I)

in welcher
- R: für Wasserstoff oder Alkyl steht, erhält,

wenn man
a) Mercaptane der allgemeinen Formel (II)

   R¹-SH (II)

   in welcher
   - R¹: für t-Butyl oder die Benzylgruppe 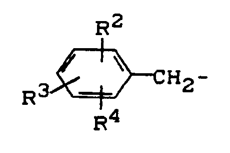 steht, in welcher
   R², R³ und R⁴ gleich oder verschieden sind und für Wasserstoff, Halogen oder Alkyl stehen, mit den Vinylidenfluoriden der allgemeinen Formel (III)

   R-CH=CF₂ (III)

   in welcher
   - R: die oben angegebenen Bedeutungen hat,
   in Gegenwart einer Base und gegebenenfalls in Gegenwart eines Lösungsmittels und gegebenenfalls in Gegenwart eines Phasentransferkatalysators, bei Temperaturen zwischen 0 und 250°C umsetzt und
b) die erhaltenen Verbindungen der allgemeinen Formel (IV)

   R¹-S-CF₂-CH₂-R (IV)

   in welcher
   R und R¹ die oben angegebenen Bedeutungen haben,
   gegebenenfalls nach ihrer Isolierung oder gegebenenfalls nach Isolierung der unter a) entstandenen Mischungen aus den Verbindungen der allgemeinen Formel (IV) und den Nebenprodukten der allgemeinen Formel (V)

   R¹-S-CF=CH-R (V)

   in welcher
   R und R¹ die oben angegebenen Bedeutungen haben,
   mit einem Chlorierungsmittel, gegebenenfalls in Gegenwart eines Lösungsmittels, bei Temperaturen zwischen -78°C und 100°C umsetzt und die erhaltenen 1,1-Difluoralkansulfenylchloride der allgemeinen Formel (I) isoliert.

Das erfindungsgemäße Verfahren ermöglicht die Herstellung der 1,1-Difluoralkansulfenylchloride der allgemeinen Formel (I) in guter Ausbeute und Reinheit. Es ist als ein technisches Verfahren zur Herstellung auch größerer Mengen dieser Verbindungen besonders geeignet. Bei Kenntnis des Standes der Technik war die glatte Durchführbarkeit des Verfahrens nicht zu erwarten, Zwar ist die nucleophile Addition von bestimmten Alkoholen an Vinylidenfluorid bekannt [R.E.A. Dear und E.E.Gilbert, J. Chem, Eng. Data 14, 493 bis 497 (1969)], die bisherigen Erfahrungen mit Mercaptoverbindungen ließen jedoch den Schluß zu, daß eine glatte Reaktion mit Mercaptoverbindungen nicht erfolgen würde.

In den allgemeinen Formeln bedeutet Alkyl geradkettiges oder verzweigtes Alkyl mit vorzugsweise 1 bis 8, insbesondere 1 bis 6 und besonders bevorzugt 1 bis 4 Kohlenstoffatomen, Vorzugsweise seien genannt: Methyl, Ethyl, n.- und i.-Propyl sowie n.-, i.-, sec.- und t.-Butyl. Besonders hervorgehoben seien Methyl und Ethyl, insbesondere Methyl.

Halogen in den allgemeinen Formeln bedeutet Fluor, Chlor, Brom und Iod, vorzugsweise Fluor, Chlor und Brom, besonders bevorzugt Fluor und Chlor und ganz besonders bevorzugt Chlor.

R steht vorzugsweise für Wasserstoff oder Methyl, besonders bevorzugt für Wasserstoff.

Die Benzylgruppe (vergl. Definition von R¹) kann 1 bis 3, vorzugsweise 1 oder 2 der angegebenen Substituenten tragen. Als bevorzugt seien 4-Methylbenzyl und 4-Chlorbenzyl angegeben. Besonders bevorzugt wird die unsubstituierte Benzylgruppe (R², R³ und R⁴ stehen für Wasserstoff).

In einer besonders bevorzugten Ausführungsform der Erfindung stehen R für Wasserstoff und R¹ für die Benzylgruppe, wobei R², R³ und R⁴ Wasserstoff bedeuten.

Ganz besonders bevorzugt wird das erfindungsgemäße Verfahren zur Herstellung von 1,1-Difluorethansulfenylchlorid (Verbindung der Formel (I) mit R = Wasserstoff) eingesetzt.

Das erfindungsgemäße Verfahren soll anhand des folgenden (nicht stöchiometrischen) Formelschemas erläutert werden:

### Stufe (a)

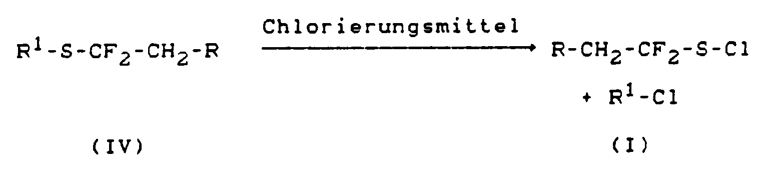

### Stufe (b)

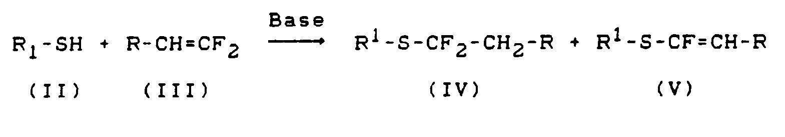

Die erfindungsgemäße Verfahrensstufe (a) wird in Gegenwart einer Base durchgeführt. Hierzu können anorganische Basen, wie Alkali- und Erdalkalimetallhydroxide, wie Natrium- und/oder Kaliumhydroxid oder Calziumhydroxid sowie organische Basen, wie Trialkylamine, z.B. Triethylamin, Amidinbasen, z.B. Diazabicycloundecen oder Diazabicyclononen verwendet werden. Auch basische Ionenaustauscher können eingesetzt werden. Vorzugsweise werden Alkalihydroxide (Natrium- und/oder Kaliumhydroxid) eingesetzt, wobei bevorzugt wäßrige Lösungen verwendet werden.

Die Base wird in wenigstens 1 molarer Menge, vorzugsweise in 1 bis 3 molarer und besonders bevorzugt in 1 bis 2 molarer Menge [bezogen auf die Verbindung der Formel (II)] eingesetzt.

Die erfindungsgemäße Verfahrensstufe (a) kann ohne Lösungsmittel oder, bevorzugt, in Gegenwart eines Lösungsmittels durchgeführt werden. Als Lösungsmittel können alle für die Reaktion inerten organischen Lösungsmittel, vorzugsweise Lösungsmittel, welche nicht oder nur wenig wassermischbar sind, eingesetzt werden. Als bevorzugte Lösungsmittel, die einzeln oder auch in Mischungen verwendet werden können, seien aufgeführt:

Aromatische Kohlenwasserstoffe, wie Benzol, Toluol und Xylole, aliphatische und cycloaliphatische Kohlenwasserstoffe, wie Petrolether und Cyclohexan, Ether, wie Di-n-butylether oder Methyl-t-butylether. Besonders bevorzugt wird Toluol verwendet.

Die erfindungsgemäße Verfahrensstufe (a) wird vorzugsweise in Gegenwart eines Phasentransferkatalysators durchgeführt, wobei alle üblichen Phasentransferkatalysatoren verwendet werden können. Vorzugsweise werden quartäre Ammoniumsalze (insbesondere Tetra-C₂-C₄-Alkyl und Benzylammoniumchloride), wie Tetra-n-butylammoniumchlorid und besonders bevorzugt Triethylbenzylammoniumchlorid (TEBA) eingesetzt.

Die Phasentransferkatalysatoren werden in Mengen von vorzugsweise 1 bis 50, insbesondere 5 bis 20 g je Liter des verwendeten Lösungsmittels eingesetzt.

Die erfindungsgemäße Verfahrensstufe (a) wird bei Temperaturen zwischen 0 und 250°C, vorzugsweise zwischen 50 und 200°C und besonders bevorzugt zwischen 80 und 120°C, durchgeführt.

Bei der erfindungsgemäßen Verfahrensstufe (a) werden je Mol Mercaptan (II) 1 bis 5 Mol, vorzugsweise 1 bis 2,5 und besonders bevorzugt 1 bis 1,5 Mol, des Vinylidenfluorides (III) eingesetzt.

Die Umsetzung der Verbindungen der Formel (II) mit den Verbindungen der Formel (III) erfolgt in einem Druckbehälter und wird bei Drücken vorzugsweise zwischen 1 und 50 bar, insbesondere zwischen 5 und 30 bar durchgeführt, wobei sich der jeweilige Druck durch das Hinzufügen (Hinzupumpen) der Vinylidenfluoride der Formel (III) sowie durch die Temperaturführung ergibt.

Die gemäß Verfahrensstufe (a) erhaltenen Reaktionsprodukte werden nach den üblichen Methoden, vorzugsweise wäßrig extraktiv isoliert und können durch Destillation gereinigt werden. Destillativ können auch die Verbindungen der Formel (IV) von den als Nebenprodukten entstehenden Verbindungen der Formel (V) abgetrennt werden. Für eine Weiterverarbeitung in der Verfahrensstufe (b) ist jedoch eine solche Abtrennung der Nebenprodukte der Formel (V) nicht erforderlich, da sie bei der weiteren Umsetzung nicht stören.

In einer bevorzugten Durchführungsform der Verfahrensstufe (a) werden die Verbindungen der Formel (II) in einem Druckgefäß in einem Lösungsmittel gelöst und mit der wäßrigen Lösung der Base sowie gegebenenfalls mit dem Phasentransferkatalysator versetzt. Bei erhöhter Temperatur werden die Verbindungen der Formel (III) innerhalb von 1 bis 18 Stunden hinzugefügt (hinzugepumpt), wobei sich ein entsprechender Druck aufbaut. Nach dem Ende der Reaktion werden die Phasen (gegebenenfalls unter Hinzufügen von Salzsäure) getrennt und aus der organischen Phase die gewünschten Verbindungen isoliert.

Die erfindungsgemäße Verfahrensstufe (b) kann mit allen für die Chlorolyse geeigneten Chlorierungsmitteln durchgeführt werden. Als geeignete und bevorzugte Chlorierungsmittel seien Sulfurylchlorid, N-Chlorsuccinimid und ganz besonders bevorzugt Chlor aufgeführt.

Die Chlorolyse (b) kann in Gegenwart von Lösungsmitteln oder, bevorzugt, ohne den Zusatz von Lösungsmitteln durchgeführt werden. Als Lösungsmittel können alle bei der Chlorierungsreaktion inerten organischen Lösungsmittel eingesetzt werden. Bevorzugt werden halogenierte (durch Fluor und/oder Chlor substituierte) Kohlenwasserstoffe, wie Tetrachlorkohlenstoff und Methylenchlorid sowie Freone bzw. Frigene verwendet.

Die Chlorierungsreaktion (b) wird bei Temperaturen zwischen -78 und 100°C, vorzugsweise zwischen -30 und 25°C und besonders bevorzugt zwischen -20 und 0°C, durchgeführt.

Bei der Chlorierungsreaktion (b) werden je Mol der Verbindungen der Formel (IV) vorzugsweise 1 bis 5 Mol, besonders bevorzugt 1 bis 3 Mol, des Chlorierungsmittels eingesetzt.

Die gewünschten Endprodukte der Formel (I) werden durch Destillation aus dem Reaktionsgemisch isoliert und gegebenenfalls destillativ weiter gereinigt.

In einer bevorzugten Ausführungsform der Verfahrensstufe (b) wird das in der Stufe (a) erhaltene Gemisch aus den Verbindungen der Formeln (IV) und (V) bei den angegebenen Temperaturen mit Chlor versetzt und der Fortgang der Reaktion analytisch durch Gaschromatographie verfolgt, Sobald keine Verbindung der Formel (IV) mehr nachgewiesen werden kann, wird das gewünschte Produkt der Formel (I) unter leichtem Vakuum durch Destillation abgetrennt.

Die erfindungsgemäß erhaltlichen Verbindungen der allgemeinen Formel (I) sind wertvolle Vor- und Zwischenprodukte für organische Synthesen, z.B. zur Herstellung von insektiziden Phosphorsaureestern (vergl. DE-A 3 930 409). So kann beispielsweise das erfindungsgemäß erhaltliche 1,1-Difluorethansulfenylchlorid mit S-s-Butyl-O,O-diethyl-thiophosphorigsauretriester in Dichlormethan als Lösungsmittel zu dem insektizid wirksamen O-Ethyl-S-s-butyl-S-(1,1-difluorethyl)-dithiophosphorsauretriester (bp. 75°C/1,3 mbar) umgesetzt werden. Dieser Phosphorsäureester kann in üblicher Weise zu einem Pflanzenschutzmittel formuliert werden, welches in üblicher Weise als Insektizid eingesetzt werden kann.

Das erfindungsgemäße Verfahren soll anhand der folgenden Beispiele erläutert werden. Wo nichts anderes angegeben wird, beziehen sich alle %-Angaben im vorliegenden Text auf Gewichtsprozente.

### Beispiel 1a [Stufe (a)]

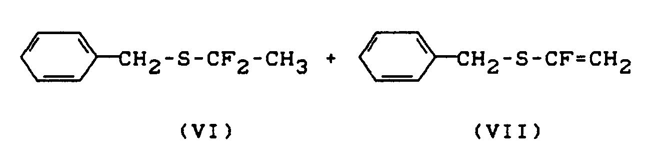

420 g (3,4 mol) Benzylmercaptan werden bei etwa 20°C in einem Autoklaven in 600 ml Toluol gelöst und mit 600 ml wäßriger 45 %iger Natronlauge und 10 g Triethylbenzylammoniumchlorid (TEBA) versetzt. Bei 100°C werden innerhalb von 12 Stunden 320 g (5 mol) Vinylidenfluorid zugepumpt, wobei der Druck von 5 auf 25 bar ansteigt. Anschließend wird 12 Stunden bei 100°C nachgerührt. Nach dem Abkühlen und Entspannen gießt man auf 2 Liter 10 %ige wäßrige Salzsäure, trennt die Phasen und extrahiert die wäßrige Phase mit Toluol. Die vereinigten organischen Phasen werden mit NaCl-Lösung gewaschen, mit MgSO₄ getrocknet und im Vakuum fraktioniert.

Man erhält 460 g (72 % Umsatz) eines 2:1 Gemisches von Benzyl-1,1-difluorethylsulfid (VI) und Benzyl-1-fluorvinylsulfid (VII) mit einem Siedebereich von 72 bis 75°C bei 2 mbar.

Das ¹H-NMR-Spektrum von (VI) (200 MHz, CDCl₃, TMS intern) zeigt charakteristische Banden bei 7.28 ppm (m, 5 H, -Ph), 4.05 ppm (s, 2 H, -CH₂-) und 1,88 ppm (t, 3 H, -CH₃-, J=16,7 Hz).

Das ¹⁹F-NMR-Spektrum von (VI) (75.4 MHz, CDCl₃, CF₃COOH ext.) zeigt ein Quartett bei 11.5 ppm (J=16.7 Hz).

Das ¹H-NMR-Spektrum von (VII) (200 MHz, CDCl₃, TMS intern) zeigt charakteristische Banden bei 7.28 ppm (m, 5 H, -Ph), 4.90 ppm (dd, 1 H, =CH, J_{H,H}=3 Hz, J_{H,F}= 12 Hz), 4.62 ppm (dd, 1 H, =CH, J_{H,H}=3 Hz, J_{H,F}=43 Hz), 3.93 ppm (s, 2 H, -CH₂-).

Das ¹⁹F-NMR-Spektrum von (VII) (75.4 MHz, CDCl₃, CF₃COOH ext.) zeigt ein Dublett von Dubletts bei -2.0 ppm (J_{H,F}= 12 Hz und 43 Hz).

### Beispiel 1b [Stufe (b)]

CH₃-CF₂-S-Cl (VIII)

326 g (1.14 mol VI / 0,66 mol VII) des Sulfidgemisches aus Beispiel 1a werden bei -15°C mit überschüssigem Chlor (177 g, 2.5 mol) versetzt, bis laut Gaschromatogramm kein (VI) mehr vorhanden ist. Anschließend wird das Produkt unter Anlegen von leichtem Vakuum abdestilliert. Man erhält 158 g (90 %) 1,1-Difluorethansulfenylchlorid (VIII) mit einem Siedepunkt von 62°C bei Normaldruck und einer Reinheit von 90 % (GC).

Das ¹H-NMR-Spektrum von (VIII) (200 MHz, CDCl₃, TMS intern) zeigt charakteristische Banden bei 2.04 ppm (t, -CH₃, J_{H,F}=16.5 Hz).

Das ¹⁹F-NMR-Spektrum von (VIII) (75.4 MHz, CDCl₃, CF₃COOH ext.) zeigt charakteristische Banden bei 6.2 ppm (q, J_{H,F}=16.5 Hz).

## Patentansprüche

1. Verfahren zur Herstellung von 1,1-Difluoralkansulfenylchloriden der allgemeinen Formel (I)
R-CH₂-CF₂-S-Cl (I)
in welcher
R für Wasserstoff oder Alkyl steht,
dadurch gekennzeichnet, daß man
a) Mercaptane der allgemeinen Formel (II)
R¹-SH (II)
in welcher
R¹ für t-Butyl oder die Benzylgruppe 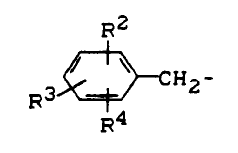 steht, in welcher
R², R³ und R⁴ gleich oder verschieden sind und für Wasserstoff, Halogen oder Alkyl stehen,
mit den Vinylidenfluoriden der allgemeinen Formel (III)
R-CH=CF₂ (III)
in welcher
R die oben angegebenen Bedeutungen hat,
in Gegenwart einer Base und gegebenenfalls in Gegenwart eines Lösungsmittels und gegebenenfalls in Gegenwart eines Phasentransferkatalysators, bei Temperaturen zwischen 0 und 250°C umsetzt und
b) die erhaltenen Verbindungen der allgemeinen Formel (IV)
R¹-S-CF₂-CH₂-R (IV)
in welcher
R und R¹ die oben angegebenen Bedeutungen haben,
gegebenenfalls nach ihrer Isolierung oder gegebenenfalls nach Isolierung der unter a) entstandenen Mischungen aus den Verbindungen der allgemeinen Formel (IV) und den Nebenprodukten der allgemeinen Formel (V)
R¹-S-CF=CH-R (V)
in welcher
R und R¹ die oben angegebenen Bedeutungen haben,
mit einem Chlorierungsmittel, gegebenenfalls in Gegenwart eines Lösungsmittels, bei Temperaturen zwischen -78°C und 100°C umsetzt und die erhaltenen 1,1-Difluoralkansulfenylchloride der allgemeinen Formel (I) isoliert.

2. Verfahren gemäß Anspruch 1, wobei R für Wasserstoff oder Alkyl steht.

3. Verfahren gemäß Anspruch 1, wobei R für Wasserstoff steht.

4. Verfahren gemäß Anspruch 1, wobei R¹ für die Benzylgruppe steht und R², R³ und R⁴ Wasserstoff bedeuten.

5. Verfahren gemäß den Ansprüchen 1 bis 4, wobei Stufe (a) in Gegenwart von Toluol durchgeführt wird.

6. Verfahren gemäß den Ansprüchen 1 bis 5, wobei in Stufe (a) als Base Natriumhydroxid eingesetzt wird.

7. Verfahren gemäß den Ansprüchen 1 bis 6, wobei in Stufe (a) als Phasentransferkatalysator Triethylbenzylammoniumchlorid verwendet wird.

8. Verfahren gemäß den Ansprüchen 1 bis 7, wobei in Stufe (b) das in Stufe (a) erhaltene Gemisch aus den Verbindungen der Formeln (IV) und (V) eingesetzt wird.

9. Verfahren gemäß den Ansprüchen 1 bis 8, wobei in Stufe (b) ohne Lösungsmittel gearbeitet wird.

10. Verfahren gemäß den Ansprüchen 1 bis 9, wobei in Stufe (b) als Chlorierungsmittel Chlor eingesetzt wird.

## Claims

1. Process for the preparation of 1,1-difluoroalkane-sulphenyl chlorides of the general formula (I)
R-CH₂-CF₂-S-Cl (I)
in which
R represents hydrogen or alkyl,
characterized in that
a) mercaptans of the general formula (II)
R¹-SH (II)
in which
R¹ represents tert-butyl or the benzyl group 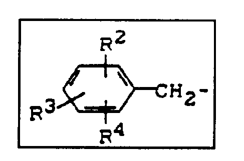 in which
R², R³ and R⁴ are identical or different and represent hydrogen, halogen or alkyl,
are reacted with the vinylidene fluorides of the general formula (III)
R-CH=CF₂ (III)
in which
R has the meanings given above
in the presence of a base and if desired in the presence of a solvent and if desired in the presence of a phase transfer catalyst, at temperatures of between 0 and 250°C, and
the resulting compounds of the general formula (IV)
R¹-S-CF₂-CH₂-R (IV)
in which
R and R¹ have the meanings given above,
are reacted, if desired after their isolation or if desired after isolation of the mixtures which were obtained in a) and comprise the compounds of the general formula (IV) and the by-products of the general formula (V)
R¹-S-CF=CH-R (V)
in which
R and R¹ have the meanings given above,
with a chlorinating agent, if desired in the presence of a solvent, at temperatures of between -78°C and 100°C, and the resulting 1,1-difluoroalkanesulphenyl chlorides of the general formula (I) are isolated.

2. Process according to Claim 1, where R represents hydrogen or alkyl.

3. Process according to Claim 1, where R represents hydrogen.

4. Process according to Claim 1, where R¹ represents the benzyl group and R², R³ and R⁴ denote hydrogen.

5. Process according to Claims 1 to 4, where step (a) is carried out in the presence of toluene.

6. Process according to Claims 1 to 5, where in step (a) the base employed is sodium hydroxide.

7. Process according to Claims 1 to 6, where in step (a) the phase transfer catalyst used is triethylbenzylammonium chloride.

8. Process according to Claims 1 to 7, where in step (b) the mixture which was obtained in step (a) and comprises the compounds of the formulae (IV) and (V) is employed.

9. Process according to Claims 1 to 8, where step (b) is carried out without a solvent.

10. Process according to Claims 1 to 9, where in step (b) the chlorinating agent employed is chlorine.

## Revendications

1. Procédé de production de chlorures de 1,1-difluoralcanesulfényle de formule générale (I)
R-CH₂-CF₂-S-Cl (I)
dans laquelle
R représente de l'hydrogène ou un groupe alkyle, caractérisé en ce qu'on fait réagir
a) des mercaptans de formule générale (II)
R¹-SH (II)
dans laquelle
R¹ est un groupe tertiobutyle ou le groupe benzyle 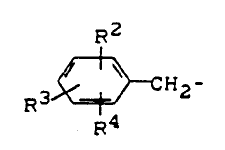 dans lequel
R², R³ et R⁴ sont identiques ou différents et représentent de l'hydrogène, un halogène ou un radical alkyle,
avec des fluorures de vinylidène de formule générale (III)
R-CH=CF₂ (III)
dans laquelle
R a les définitions indiquées ci-dessus, à des températures comprises entre 0 et 250°C en présence d'une base et le cas échéant en présence d'un solvant, et en la présence éventuelle d'un catalyseur de transfert de phases, et
b) on fait réagir les composés obtenus de formule générale (IV)
R¹-S-CF₂-CH₂-R (IV)
dans laquelle
R et R¹ ont les définitions indiquées ci-dessus,
le cas échéant après les avoir isolés ou éventuellement après isolement des mélanges produits en a) des composés de formule générale (IV) et des sous-produits de formule générale (V)
R¹-S-CF=CH-R (V)
dans laquelle
R et R¹ ont les définitions indiquées ci-dessus,
avec un agent de chloration, éventuellement en présence d'un solvant, à des températures comprises entre -78°C et 100°C et on isole les chlorures de 1,1-difluoralcanesulfényle obtenus, de formule générale (I).

2. Procédé suivant la revendication 1, dans lequel R représente de l'hydrogène ou un groupe alkyle.

3. Procédé suivant la revendication 1, dans lequel R représente de l'hydrogène.

4. Procédé suivant la revendication 1, dans lequel R¹ représente le groupe benzyle et R², R³ et R⁴ désignent de l'hydrogène.

5. Procédé suivant les revendications 1 à 4, dans lequel l'étape (a) est conduite en présence de toluène.

6. Procédé suivant les revendications 1 à 5, dans lequel on utilise comme base dans l'étape (a) l'hydroxyde de sodium.

7. Procédé suivant les revendications 1 à 6, dans lequel on utilise comme catalyseur de transfert de phases dans l'étape (a) le chlorure de triéthylbenzylammonium.

8. Procédé suivant les revendications 1 à 7, dans lequel on utilise dans l'étape (b) le mélange obtenu dans l'étape (a) des composés de formules (IV) et (V).

9. Procédé suivant les revendications 1 à 8, dans lequel on opère en l'absence du solvant dans l'étape (b).

10. Procédé suivant les revendications 1 à 9, dans lequel on utilise le chlore comme agent de chloration dans l'étape (b).
